# EUROPEAN PATENT SPECIFICATION

(11) **EP 0 263 005 B1**
(45) Date of publication and mention of the grant of the patent: **23.08.1995**
(21) Application number: 87401987.0
(22) Date of filing: 04.09.1987
(51) Int. Cl.: B01J 27/28, C07C 51/377, C07C 57/04

(54) **Oxidative dehydrogenation of saturated organic compounds in the presence of regenerated catalysts**
Verfahren zur oxydativen Dehydrierung von gesättigten organischen Verbindungen in Gegenwart von regenerierten Katalysatoren
Procédé de déshydrogénation par oxydation de composés organiques saturés en présence de catalyseurs régénérés

(30) Priority: 09.09.1986 GB 8621703
(43) Date of publication of application: 06.04.1988
(73) Proprietor: ELF ATOCHEM S.A., 92800 Puteaux (FR)
(72) Inventor: Scaccia, Carlo, Worthington Ohio 43085 (US); Eckler, James H., Dublin Ohio 43017 (US)
(74) Representative: Rochet, Michel

(56) References cited:
- EP-A- 0 123 467
- FR-A- 2 498 476
- US-A- 3 716 545
- US-A- 4 515 899

## Description

The present invention relates to a process for the oxidative dehydrogenation of satured organic compounds in the presence of at least one modified iron phophate catalyst.

There is already known, namely through French Application No 2 497 795, a process for the catalytic conversion of isobutyric acid to methacrylic acid via the oxydehydrogenation reaction wherein an iron phosphate catalyst is contacted with a gaseous feed stream containing said acid substrate and oxygen at a temperature between about 300 and 550°C. It is also known that such iron phosphate catalysts tend to deactivate after being on-stream for a few hundreds of hours. In view to overcome this disadvantage there have been designed methods of regenerating iron phosphate catalysts. For example British Patent No 2 097 690 discloses a method of regenerating an iron phosphate catalyst used in the dehydrogenation of a feed stream of an organic compound comprising the steps of:
a) discontinuing said feed stream and subjecting the catalyst to an oxygen-containing oxidizing atmosphere at a temperature of 350°C or greater for at least two hours; and
b) then subjecting the catalyst to a mildly reducing atmosphere comprising said feed stream organic compound at 350°C or greater.

However this method suffers the two following disadvantages :
- used catalyst can only be partially regenerated, and
- after regeneration, nearly fresh catalyst activity is attained but the rate of subsequent deactivation is greatly increased.

These disadvantages may be due to local temperature surges causing melting of some of the catalyst present, or to some changes in the catalyst surface due to a secondary deactivation mechanism. Whatever the cause may be, it was observed that the catalyst did not return to the original selectivity and conversion after being regenerated by such a method. This is why the aim of the present invention is to provide a method of regenerating an iron phosphate catalyst which would satisfy both requirements of a complete regeneration without increasing the rate of subsequent deactivation after regeneration. Another aim of the present invention is to provide a process for the oxidative dehydrogenation of saturated organic compound, using an iron phosphate catalyst, whereby by means of a suitable method for regenerating the said catalyst, the oxidative dehydrogenation reaction could be performed while using the same catalyst for at least about 1,000 hours.

Therefore a first object of the present invention is a process for the oxidative dehydrogenation of a compound having the formula: 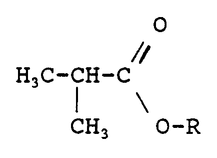
wherein R is selected from the group consisting of the hydrogen atom and linear or branched saturated aliphatic groups having from 1 to 8 carbon atoms, in the presence of at least one modified iron phosphate catalyst having the gram-atom empirical formula FePₓM_{y}O_{z} wherein:
- M is at least one metal selected from the group consisting of lithium, sodium, rubidium, cesium, magnesium, calcium, strontium, baryum, cobalt, lanthane, tellurium and silver,
- x is not below 0.2 but not above 2,
- y is not below 0.01 but not above 2, and
- z is the amount of oxygen linked to the other elements and corresponding to their oxidation stage,
characterized in that the said catalyst is regenerated, intermittently or continuously, by the addition thereto of an effective amount of at least one phosphorus-containing compound. The regeneration process according to the present invention may be effected either batchwise or continuously, depending on the economic conditions of the chemical process wherein the modified iron phosphate catalyst is used. The effective amount of the phosphorus-containing compound to be added to the modified iron phosphate catalyst will most generally depend on the temperature at which the said catalyst is used. The rate of deactivation of the catalyst is greater at higher temperatures ; consequently, a greater regeneration chemical flow is needed to maintain constant conversion and selectivity at higher temperature. Similarly it should be noted that the catalyst subjected to the regeneration process of the present invention should preferably be in the form of particles having a mean particle size of from about 100 »m to 15 mm.

Phosphoric acid H₃PO₄ may be used as the phosphorus-containing compound to add to the modified iron phosphate catalyst. Phosphoric acid may be used in the process according to the present invention in the form of an aqueous solution. However, while phosphoric acid is an effective way of maintaining constant catalyst activity, phosphoric acid presents the well-known problem of corrosion of stainless steel equipment. To eliminate this problem, organo phosphates like tributyl phosphate or triethyl phosphate may be used preferably as the phosphorus-containing compound to add to the modified iron phosphate catalyst.

When the regenaration process according to the present invention is effected batchwise and the modified iron phosphate catalyst is used at 415°C, an efficient regeneration of the said catalyst will be achieved while using the phosphorus-containing compound in a weight ratio of from about 0.05 : 1 to about 0.5 : 1 with respect to the catalyst. In such case, complete regeneration of the catalyst will generally be performed within a time ranging from about 6 (six) to 60 (sixty) hours.

When the regeneration process according to the present invention is effected continuously and the modified iron phosphate catalyst is used at 415°C, an efficient regeneration of the said catalyst will most generally be achieved while using the phosphorus-containing compound in a ratio of from about 200 to about 1,600 mg per kg of catalyst and per hour.

A specific example of the compound of the above-mentioned formula is isobutyric acid which may be accordingly converted to methacrylic acid. In this reaction, the isobutyric acid in a gaseous mixture with oxygen from air and one or more diluents such as nitrogen, steam or carbon dioxide is passed through the reaction chamber wherein the iron phosphate catalyst is contained. As an example, the reactor is a tubular reactor, but other reaction vessels can be used as well. The amount of saturated acid contained in the reaction mixture is generally 1 to 35 % by volume and preferably 5 to 10 % by volume. The reaction is preferably conducted using a fixed catalytic bed and is conducted at a temperature from about 300°C to 500°C and, preferably, from 380°C to 450°C. The contact time expressed in seconds is the ratio between the volume of the catalytic bed and the volume of the gaseous mixture reagents fed per second under normal conditions (atmospheric pressure, 0°C). The average conditions of temperature and pressure existing in a bed may vary depending upon the nature of the catalyst, the nature of the catalytic bed, and upon the catalyst size.

Generally, the contact time is from 0.1 to 20 seconds and, preferably, 0.3 to 15 seconds.

At least in the case of this particular embodiment of the invention, a complete and satisfactory regeneration of the catalyst may be achieved by means of the present invention without suffering any disadvantage with respect to the isobutyric acid conversion and/or the methacrylic acid selectivity. For example both conversion and selectivity may be maintained for as long as 10,000 hours.

The following examples are designed to illustrate the present invention without limiting the scope thereof in any way.

### Example 1 (comparative)

Oxidative dehydrogenation of isobutyric acid into methacrylic acid was effected at 415°C using a mixture of isobutyric acid, oxygen and water in a tubular reactor containing a modified iron phosphate catalyst having the gram-atom empirical formula FeP₁ ₂₃Cs ₁₅Oₓ and a particle size of 1.2 mm. Molten salts were used to maintain reactor wall temperature. The molar ratio of water to isobutyric acid is 25 : 1 and the molar ratio of oxygen to isobutyric acid is 0.75 : 1. There is observed a 97 % isobutyric acid conversion and a 79 % methacrylic acid selectivity when the catalyst is fresh. In the absence of any regeneration of the catalyst, conversion falls down to 67 % and selectivity falls down to 46 % after 3,000 hours on stream.

### Example 2

After 3,000 hours on stream of the oxidative dehydrogenation reaction effected according to example 1, the water feed to the reactor is replaced with a 7,000 ppm phosphoric acid aqueous solution (36,000 ppm, based on the isobutyric acid flow). After about 6 hours, the peak reaction temperature begins to fall, coinciding with a drop in isobutyric acid conversion. One hour after the peak reaction temperature is within 1°C of the molten salt (reactor wall) temperature, the phosphoric acid aqueous solution was replaced with the original deionized water feed. During the next 60 hours, isobutyric acid conversion continues to climb up to a maximum of 96 % whereas methacrylic acid selectivity reaches a value of 82 %.

### Example 3

After the catalyst regeneration successfully performed according to example 2, the same catalyst can be regenerated several times again under the same conditions. Even after the 14th regeneration at 9,300 hours on stream, isobutyric acid conversion was 92 % and methacrylic acid selectivity was 79 %.

### Example 4 (comparative)

Oxidative dehydrogenation of isobutyric acid into methacrylic acid was effected at 400°C using a mixture of isobutyric acid, oxygen and water in a bench scale integral reactor containing the modified iron phosphate catalyst described in example 1. The molar ratio of water to isobutyric acid is 15 : 1 and the molar ratio of oxygen to isobutyric acid is 0.75 : 1. There is achieved a 92 % isobutyric acid conversion when the catalyst is fresh. In the absence of any regeneration of the catalyst, conversion falls down to 82 % after 1,000 hours on stream.

### Example 5

Continuous regeneration of the catalyst used under the conditions of example 4 is effected by co-feeding 100 ppm phosphoric acid (based on water) to the reactor. Isobutyric acid conversion is thus maintained constant at the level of 92 % for at least 1,000 hours on stream.

### Example 6

Continuous regeneration of the catalyst used under the conditions of example 4 (slightly modified by using a molar ratio of oxygen to isobutyric acid equal to 0.65 : 1 and a reaction temperature of 415°C) is effected by co-feeding 300 ppm tributyl phosphate (based on the isobutyric acid feed) to the reactor. Isobutyric acid conversion may thus be maintained constant for at least 1,800 hours on stream.

## Claims

1. A process for the oxidative dehydrogenation of a compound having the formula : 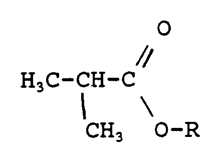 wherein R is selected from the group consisting of the hydrogen atom and linear or branched saturated aliphatic groups having from 1 to 8 carbon atoms,
in the presence of at least one modified iron phosphate catalyst having the gram-atom empirical formula FePₓM_{y}O_{z} wherein :
- M is at least one metal selected from the group consisting of lithium, sodium, rubidium, cesium, magnesium, calcium, strontium, baryum, cobalt, lanthane, tellurium and silver,
- x is not below 0.2 but not above 2,
- y is not below 0.01 but not above 2, and
- z is the amount of oxygen linked to the other elements and corresponding to their oxidation stage,
characterized in that the said catalyst is regenerated, intermittently or continuously, by the addition thereto of an effective amount of at least one phosphorus-containing compound.

2. A process according to claim 1, characterized in that the said compound is isobutyric acid.

3. A process according to one of claims 1 and 2, characterized in that the said phosphorus-containing compound is phosphoric acid H₃PO₄.

4. A process according to one of claims 1 and 2, characterized in that the said phosphorus-containing compound is an organo phosphate.

5. A process according to any of claims 1 to 4, characterized in that it is effected batchwise.

6. A process according to claim 5, characterized in that the phosphorus-containing compound is in a weight ratio of from 0.05 : 1 to 0.5 : 1 with respect to the modified iron phosphate catalyst when such catalyst is used at 415°C.

7. A process according to any of claims 1 to 4, characterized in that it is effected continuously.

8. A process according to claim 7, characterized in that the phosphorus-containing compound is used at the rate of from 200 to 1,600 mg per kg of catalyst and per hour.

## Patentansprüche

1. Verfahren zur oxidativen Dehydrierung einer Verbindung mit der Formel 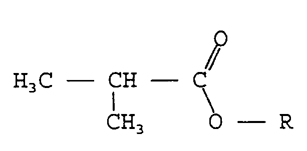 worin R aus der aus dem Wasserstoffatom und linearen oder verzweigten gesättigten aliphatischen Gruppen mit 1 bis 8 Kohlenstoffatomen bestehenden Gruppe gewählt wird,
in der Gegenwart wenigstens eines modifizierten Eisenphosphatkatalysators mit der empirischen Grammatomformel FePₓM_{y}O_{z}, worin:
- M wenigstens ein aus der aus Lithium, Natrium, Rubidium, Cäsium, Magnesium, Calcium, Strontium, Barium, Kobalt, Lanthan, Tellur und Silber bestehenden Gruppe gewähltes Metall ist,
- x nicht unter 0,2, jedoch nicht über 2 ist,
- y nicht unter 0,01, jedoch nicht über 2 ist und
- z die Menge von mit den anderen Elementen verbundenem und ihrer Oxidationsstufe entsprechendem Sauerstoff ist,
dadurch gekennzeichnet, daß
der Katalysator diskontinuierlich oder kontinuierlich durch den Zusatz einer wirksamen Menge wenigstens einer phosphorhaltigen Verbindung regeneriert wird.

2. Verfahren nach Anspruch 1, dadurch gekennzeichnet, daß die Verbindung Isobutyrsäure ist.

3. Verfahren nach einem der Ansprüche 1 und 2, dadurch gekennzeichnet, daß die phorphorhaltige Verbindung Phosphorsäure H₃PO₄ ist.

4. Verfahren nach einem der Ansprüche 1 und 2, dadurch gekennzeichnet, daß die phosphorhaltige Verbindung ein Organophosphat ist.

5. Verfahren nach irgendeinem der Ansprüche 1 bis 4, dadurch gekennzeichnet, daß es schubweise durchgeführt wird.

6. Verfahren nach Anspruch 5, dadurch gekennzeichnet, daß die phosphorhaltige Verbindung in einem Gewichtsverhältnis von 0,05 : 1 bis 0,5 : 1 zum modifizierten Eisenphosphatkatalysator steht, wenn dieser Katalysator bei 415 °C verwendet wird.

7. Verfahren nach irgendeinem der Ansprüche 1 bis 4, dadurch gekennzeichnet, daß es kontinuierlich durchgeführt wird.

8. Verfahren nach Anspruch 7, dadurch gekennzeichnet, daß die phosphorhaltige Verbindung bei einem Durchsatz von 200 bis 1600 mg je kg des Katalysators und je Stunde verwendet wird.

## Revendications

1. Procédé de déshydrogénation par oxydation d'un composé de formule: 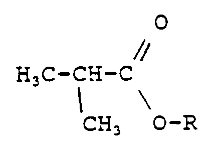 dans laquelle R est choisi dans le groupe constitué par l'atome d'hydrogène et des groupes aliphatiques saturés, linéaires ou ramifiés, comportant de 1 à 8 atomes de carbone, en présence d'au moins un catalyseur modifié à base de phosphate de fer de formule globale empirique FePₓM_{y}O_{z}, dans laquelle :
- M représente au moins un métal choisi dans le groupe constitué par le lithium, le sodium, le rubidium, le césium, le magnésium, le calcium, le strontium, le baryum, le cobalt, le lanthane, le tellure et l'argent,
- x n'est pas inférieur à 0,2, mais pas supérieur à 2,
- y n'est pas inférieur à 0,01, mais pas supérieur à 2, et
- z est la quantité d'oxygène lié aux autres éléments et correspondant à leur état d'oxydation,
caractérisé en ce que ledit catalyseur est régénéré, par intermittence ou en continu, par addition à ce dernier d'une quantité efficace d'au moins un composé phosphoré.

2. Procédé selon la revendication 1, caractérisé en ce que ledit composé est l'acide isobutyrique.

3. Procédé selon l'une quelconque des revendications 1 et 2, caractérisé en ce que ledit composé phosphoré est l'acide phosphorique H₃PO₄.

4. Procédé selon l'une quelconque des revendications 1 et 2, caractérisé en ce que ledit composé phosphoré est un organo-phosphate.

5. Procédé selon l'une quelconque des revendications 1 à 4, caractérisé en ce qu'il est effectué en discontinu.

6. Procédé selon la revendication 5, caractérisé en ce que le composé phosphoré est présent dans un rapport pondéral de 0,05:1 à 0,5:1 par rapport au catalyseur modifié à base de phosphate de fer, lorsque ce catalyseur est utilisé à 415°C.

7. Procédé selon l'une quelconque des revendications 1 à 4, caractérisé en ce qu'il est effectué en continu.

8. Procédé selon la revendication 7, caractérisé en ce que le composé phosphoré est utilisé à raison de 200 à 1 600 mg par kg de catalyseur et par heure.
